# EUROPEAN PATENT APPLICATION

(11) **EP 4 375 375 A1**
(43) Date of publication of application: **29.05.2024**
(21) Application number: 22845877.4
(22) Date of filing: 15.07.2022
(51) Int. Cl.: C12N 15/11, C12Q 1/6851, C12Q 1/686, G01N 33/53, G01N 33/574, C07K 14/705

(54) **BIOMARKER FOR PREDICTING PROGNOSIS OF CANCER PATIENT, METHOD FOR PREDICTING PROGNOSIS OF CANCER PATIENT, METHOD FOR PREDICTING EFFECT OF CANCER THERAPEUTIC DRUG ON CANCER PATIENT, AND KIT FOR PREDICTING PROGNOSIS OF CANCER PATIENT**

(30) Priority: 20.07.2021 JP 2021119757
(71) Applicant: Tohoku University, Sendai-shi, Miyagi 980-8577 (JP)
(72) Inventor: TAKAI, Toshiyuki, Sendai-shi, Miyagi 980-8577 (JP); OKADA, Yoshinori, Sendai-shi, Miyagi 980-8577 (JP); KUMATA, Sakiko, Sendai-shi, Miyagi 980-8577 (JP); SU, Mei tzu, Sendai-shi, Miyagi 980-8577 (JP); ENDO, Shota, Sendai-shi, Miyagi 980-8577 (JP); NOTSUDA, Hirotsugu, Sendai-shi, Miyagi 980-8577 (JP); TANAKA, Ryota, Sendai-shi, Miyagi 980-8577 (JP); SAITO, Ryoko, Sendai-shi, Miyagi 980-8577 (JP)
(74) Representative: Plasseraud IP
(86) International application number: PCT/JP2022/027853
(87) International publication number: WO 2023/002943

(57) **Abstract**

A biomarker for predicting prognosis of a cancer patient, including an immunosuppressive receptor LILRB4 or an immunosuppressive receptor LILRB4 gene; a method for predicting prognosis of a cancer patient including measuring an expression level of an immunosuppressive receptor LILRB4 or an immunosuppressive receptor LILRB4 gene in a biological sample of a cancer patient; a method for predicting an effect of a substance, as a cancer therapeutic drug, that inhibits binding between fibronectin and an immunosuppressive receptor LILRB4 in a cancer patient, the method including measuring an expression level of an immunosuppressive receptor LILRB4 or an immunosuppressive receptor LILRB4 gene in a biological sample of a cancer patient; and a kit for predicting prognosis of a cancer patient including a reagent for measuring an expression level of the biomarker.

## Description

### TECHNICAL FIELD

The present invention relates to a biomarker for predicting prognosis of a cancer patient, a method for predicting prognosis of a cancer patient, a method for predicting an effect of a cancer therapeutic drug in a cancer patient, and a kit for predicting prognosis of a cancer patient.

Priority is claimed based on Japanese Patent Application No. 2021-119757, filed July 20, 2021, the content of which is incorporated herein.

### BACKGROUND ART

In recent years, cancer immunotherapies using immune checkpoint inhibitors have attracted attention. The immune checkpoint inhibitor is an agent that binds to an immune checkpoint molecule, which has an action of suppressing an autoimmune response and suppressing an excessive immune reaction, or a ligand thereof to inhibit transmission of an immunosuppressive signal, and thereby T cells and other immune system cells are released from the immune checkpoint molecule's suppression and activated. In cancer immunotherapies, the immune checkpoint inhibitor binds to an immune checkpoint molecule having an action of suppressing an immune response against a tumor or a ligand thereof to reduce transmission of an immunosuppressive signal, and thereby T cells and other immune system cells are released from the immune checkpoint molecule's suppression and activated, and thus an antitumor action is exhibited.

Patent Document 1 reports that inflammatory diseases derived from infections or autoimmunity can be determined by using LILRB4 (leukocyte Ig-like receptor B4, hereinafter also referred to as B4), which is an immunosuppressive receptor for an unknown ligand.

Patent Document 2 reports that a physiological ligand of B4 is fibronectin, and that a substance that inhibits binding between B4 and the fibronectin is effective for treatment of immune checkpoint-associated diseases.

Regarding B4, it has been reported that B4 helps tumor cells to infiltrate tissues, that a B4-knockout cell strain of tumor cells suppresses development of acute leukemias, and that tumor-bearing mice of the B4-knockout cell strain have better survival rates than tumor-bearing mice of the parent strain (Non-Patent Document 1).

Tumor infiltrating lymphocytes (hereinafter, also referred to as TILs) are lymphocytes found in tumors, and it has been reported that an increase in number of TILs in cancer tissues significantly correlates with good prognosis in groups of non-small cell lung carcinoma patients of stages I to III (Non-Patent Document 2).

With regard to the relationship between expression of an immunosuppressive receptor on TILs and prognosis of cancer, there is a report that patients positive for expression of an immunosuppressive receptor PD-1 on TILs have poor prognosis in renal cell carcinoma (Non-Patent Document 3), but there is also a report that the expression level of PD-1 on TILs did not affect an overall survival rate and recurrence-free survival rate (Non-Patent Document 4). Whether the expression of the immunosuppressive receptor on TILs is related to the prognosis of cancer has not yet been determined.

### Citation List

### Patent Literature

Patent Document 1: JP 2018-25554 A
Patent Document 2: WO 2021/029318

### Non-Patent Document

Non-Patent Document 1: Nature. 2018 Oct; 562 (7728): 605 to 609.
Non-Patent Document 2: Hum Pathol. 2018. 79, 188-198
Non-Patent Document 3: J Clin Med. 2019.24; 8: 743.
Non-Patent Document 4: Onco Targets Ther. 2020.9; 13: 215-224.

### SUMMARY OF INVENTION

### Technical Problem

The present invention relates to a biomarker for predicting prognosis of a cancer patient, including LILRB4, a method for predicting prognosis of a cancer patient, a method for predicting an effect of a cancer therapeutic drug in a cancer patient, and a kit for predicting prognosis of a cancer patient.

### Solution to Problem

The present inventors have found that LILRB4 or LILRB4 genes are useful as biomarkers for predicting prognosis of cancer patients, and completed the present invention.

The present invention includes the following aspects.
[1] A biomarker for predicting prognosis of a cancer patient, the biomarker including an immunosuppressive receptor LILRB4 or an immunosuppressive receptor LILRB4 gene.
[2] The biomarker according to [1], where the cancer is lung cancer.
[3] A method for predicting prognosis of a cancer patient, the method including:
   measuring an expression level of an immunosuppressive receptor LILRB4 or an immunosuppressive receptor LILRB4 gene in a biological sample of the cancer patient,
   where
   a higher expression level of the immunosuppressive receptor LILRB4 or the immunosuppressive receptor LILRB4 gene in tumor infiltrating immune system cells in the biological sample than an expression level of the immunosuppressive receptor LILRB4 or the immunosuppressive receptor LILRB4 gene in a control indicates poor prognosis of the cancer patient.
[4] The method according to [3], where the cancer is lung cancer.
[5] The method according to [3] or [4], where in the measuring of the expression level of the immunosuppressive receptor LILRB4, the expression level of the immunosuppressive receptor LILRB4 is measured using an anti-immunosuppressive receptor LILRB4 monoclonal antibody or a fragment thereof.
[6] The method according to any one of [3] to [5], where
   an amino acid sequence of a complementarity determining region (hereinafter, also referred to as CDR) 1 of a heavy chain variable region (hereinafter, also referred to as VH) of the anti-immunosuppressive receptor LILRB4 monoclonal antibody contains an amino acid sequence represented by SEQ ID NO: 3,
   an amino acid sequence of a CDR2 of the VH contains an amino acid sequence represented by SEQ ID NO: 4,
   an amino acid sequence of a CDR3 of the VH contains an amino acid sequence represented by SEQ ID NO: 5,
   an amino acid sequence of a CDR1 of a light chain variable region (hereinafter, also referred to as VL) contains an amino acid sequence represented by SEQ ID NO: 6,
   an amino acid sequence of a CDR2 of the VL contains an amino acid sequence represented by SEQ ID NO: 7, and
   an amino acid sequence of a CDR3 of the VL contains an amino acid sequence represented by SEQ ID NO: 8.
[7] The method according to any one of [3] to [6], where the cancer patient is a cancer patient who has been administered a cancer therapeutic drug.
[8] The method according to [7], where the cancer therapeutic drug is a substance that inhibits binding between fibronectin and the immunosuppressive receptor LILRB4.
[9] The method according to [8], where the substance that inhibits binding between fibronectin and the immunosuppressive receptor LILRB4 is an anti-immunosuppressive receptor LILRB4 antibody.
[10] A method for predicting an effect, as a cancer therapeutic drug in a cancer patient, of a substance that inhibits binding between fibronectin and an immunosuppressive receptor LILRB4, the method including:
   measuring an expression level of an immunosuppressive receptor LILRB4 or an immunosuppressive receptor LILRB4 gene in a biological sample of the cancer patient,
   where
   a higher expression level of the immunosuppressive receptor LILRB4 or the immunosuppressive receptor LILRB4 gene in tumor infiltrating immune system cells in the biological sample than an expression level of the immunosuppressive receptor LILRB4 or the immunosuppressive receptor LILRB4 gene in a control indicates that the cancer therapeutic drug has an effect on the cancer patient.
[11] The method according to [10], where the cancer is lung cancer.
[12] The method according to [10] or [11], where the substance that inhibits binding between fibronectin and an immunosuppressive receptor LILRB4 is an anti-immunosuppressive receptor LILRB4 antibody.
[13] The method according to any one of [10] to [12], where in the measuring of the expression level of the immunosuppressive receptor LILRB4, the expression level of the immunosuppressive receptor LILRB4 is measured using an anti-immunosuppressive receptor LILRB4 monoclonal antibody or a fragment thereof.
[14] The method according to [13], where
   an amino acid sequence of a complementarity determining region (hereinafter, also referred to as CDR) 1 of a heavy chain variable region (hereinafter, also referred to as VH) of the anti-immunosuppressive receptor LILRB4 monoclonal antibody contains an amino acid sequence represented by SEQ ID NO: 3,
   an amino acid sequence of a CDR2 of the VH contains an amino acid sequence represented by SEQ ID NO: 4,
   an amino acid sequence of a CDR3 of the VH contains an amino acid sequence represented by SEQ ID NO: 5,
   an amino acid sequence of a CDR1 of a light chain variable region (hereinafter, also referred to as VL) contains an amino acid sequence represented by SEQ ID NO: 6,
   an amino acid sequence of a CDR2 of the VL contains an amino acid sequence represented by SEQ ID NO: 7, and
   an amino acid sequence of a CDR3 of the VL contains an amino acid sequence represented by SEQ ID NO: 8.
[15] A kit for predicting prognosis of a cancer patient, the kit including a reagent for measuring an expression level of the biomarker described in [1] or [2].
[16] The kit according to [15], where the cancer is lung cancer.
[17] The method according to [15] or [16], where the reagent for measuring an expression level of the biomarker includes an anti-immunosuppressive receptor LILRB4 monoclonal antibody or a fragment thereof.
[18] The kit according to [17], where
   an amino acid sequence of a complementarity determining region (hereinafter, also referred to as CDR) 1 of a heavy chain variable region (hereinafter, also referred to as VH) of the anti-immunosuppressive receptor LILRB4 monoclonal antibody contains an amino acid sequence represented by SEQ ID NO: 3,
   an amino acid sequence of a CDR2 of the VH contains an amino acid sequence represented by SEQ ID NO: 4,
   an amino acid sequence of a CDR3 of the VH contains an amino acid sequence represented by SEQ ID NO: 5,
   an amino acid sequence of a CDR1 of a light chain variable region (hereinafter, also referred to as VL) contains an amino acid sequence represented by SEQ ID NO: 6,
   an amino acid sequence of a CDR2 of the VL contains an amino acid sequence represented by SEQ ID NO: 7, and
   an amino acid sequence of a CDR3 of the VL contains an amino acid sequence represented by SEQ ID NO: 8.

### Advantageous Effects of Invention

The present invention can provide a biomarker for predicting prognosis of a cancer patient, including LILRB4 or LILRB4 gene, a method for predicting prognosis of a cancer patient, a method for predicting an effect of a cancer therapeutic drug in a cancer patient, and a kit for predicting prognosis of a cancer patient.

### BRIEF DESCRIPTION OF DRAWINGS

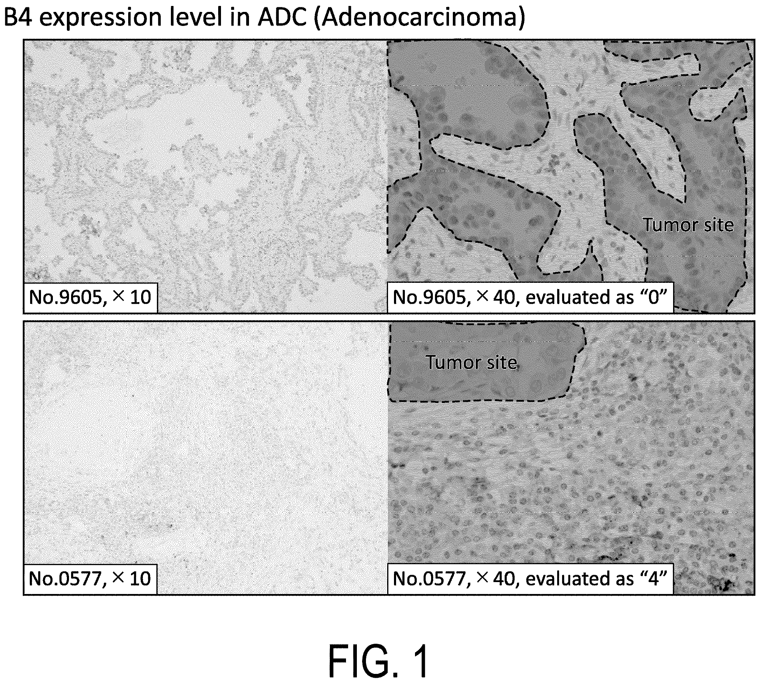

FIG. 1 illustrates representative examples of LILRB4 immunohistochemical staining of a TIL region in a lung adenocarcinoma patient in Experimental Example 4. An upper figure (No. 9605) illustrates a photomicrograph of immunohistochemical staining of a TIL region with low LILRB4 expression, and a lower figure (No.0577) illustrates a photomicrograph of immunohistochemical staining of a TIL region with high LILRB4 expression. A portion surrounded by a dotted line is a tumor portion, and portions other than the portion indicate the TIL regions. In both the upper and lower figures, a left figure illustrates a 10-fold magnified photograph, and a right figure illustrates a 40-fold magnified photograph.
FIG. 2A illustrates overall survival rates of a high LILRB4 expression group and a low LILRB4 expression group in Experimental Example 5.
FIG. 2B illustrates recurrence-free survival rates of the high LILRB4 expression group and the low LILRB4 expression group in Experimental Example 5.

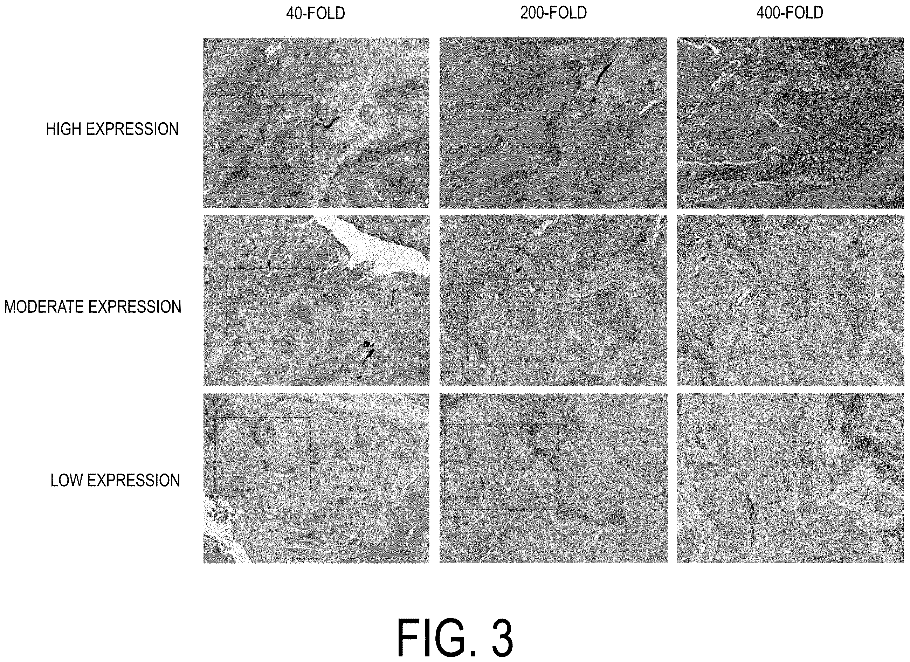

FIG. 3 illustrates representative examples of LILRB4 immunohistochemical staining of a TIL region in a lung squamous cell carcinoma patient in Experimental Example 7. An upper figure illustrates a photomicrograph of immunohistochemical staining of a TIL region with high LILRB4 expression, a middle figure illustrates a photomicrograph of immunohistochemical staining of a TIL region with moderate LILRB4 expression, and a lower figure shows a photomicrograph of immunohistochemical staining of a TIL region with low LILRB4 expression. A portion surrounded by a dotted line illustrates an enlarged portion. It should be noted that the upper, middle, and lower figures each illustrate 40-fold, 200-fold, and 400-fold magnified photographs from the left.
FIG. 4A illustrates overall survival rates of a high LILRB4 expression group and a low LILRB4 expression group in Experimental Example 9.
FIG. 4B illustrates recurrence-free survival rates of the high LILRB4 expression group and the low LILRB4 expression group in Experimental Example 9.
FIG. 5A illustrates overall survival rates of a high LILRB4 expression group and a low LILRB4 expression group in Experimental Example 10.
FIG. 5B illustrates recurrence-free survival rates of the high LILRB4 expression group and the low LILRB4 expression group in Experimental Example 10.

### DESCRIPTION OF EMBODIMENTS

### Biomarker for Predicting Prognosis of Cancer Patient

In one embodiment, the present invention provides a biomarker for predicting prognosis of a cancer patient, and the biomarker includes an immunosuppressive receptor LILRB4 or LILRB4 gene.

The base sequence and amino acid sequence of LILRB4 can be found in the database provided by the National Center for Biotechnology Information (NCBI). In a case of human (Homo sapiens) LILRB4, for example, Entrez Gene ID is 11006 (as of June 17, 2019), and RefSeq Protein IDs are NP_001265355.2, NP_001265356.2, NP_001265357.2, NP_001265358.2, and NP_001265359.2 (corresponding to isoforms 1 to 5). In a case of mouse (Mus musculus) LILRB4, for example, Gene ID is 14728 (as of June 24, 2016) and RefSeq Protein ID is NP_038560.1; in a case of rat (Rattus norvegicus) LILRB4, for example, Gene ID is 292594 (as of April 18, 2019) and RefSeq Protein ID is NP_001013916; and other animals are known to have LILRB4.

As will be described in the Examples below, the present inventors have found that cancer patients with high expression levels of LILRB4 or LILRB4 genes in tumor infiltrating immune system cells including TILs have poor prognosis.

Therefore, the marker for predicting prognosis of a cancer patient according to the present embodiment is useful for prediction of the prognosis of a cancer patient or as an index for appropriately selecting a cancer treatment strategy. For example, it is judged whether or not patients are to be applied with anti-PD-1 inhibitory antibodies or anti-PD-L1 inhibitory antibodies, by measuring expression of PD-1 or PD-L1 on cancer tissues of cancer patients. There is a possibility that the efficacy of the anti-PD-1 inhibitory antibodies or PD-L1 inhibitory antibodies cannot be expected for patients with low expression levels of PD-1 or its ligand PD-L1 in cancer tissues. However, as will be described in the Examples below, it has been found that patients with high expression levels of LILRB4 or LILRB4 genes in tumor infiltrating immune system cells of cancer patients have poor prognosis. Therefore, since cancer patients with high expression of LILRB4 or LILRB4 genes on tumor infiltrating immune system cells are judged to have poor prognosis, it is judged to be preferable to select substances that inhibit binding between fibronectin and the immunosuppressive receptor LILRB4, such as anti-LILRB4 inhibitory antibodies, for patients with high expression of LILRB4 or LILRB4 genes on tumor infiltrating immune system cells.

In the biomarker for predicting prognosis of a cancer patient according to the present embodiment, the LILRB4 or LILRB4 genes are preferably those of humans for the prediction of the prognosis of a cancer patient. That is, the biomarker for predicting prognosis of a cancer patient according to the present embodiment is preferably human LILRB4 or human LILRB4 genes.

In the biomarker for predicting prognosis of a cancer patient according to the present embodiment, the LILRB4 genes may be mRNAs of the LILRB4 genes or cDNAs obtained by reverse transcription of the mRNAs of the LILRB4 genes.

In addition, the cancer targeted by the biomarker for predicting prognosis of cancer of a cancer patient according to the present embodiment is not particularly limited, and examples thereof include solid cancer, more specifically, lung cancer, breast cancer, liver cancer, colon cancer, and malignant melanoma. The biomarker is preferably used for lung cancer. The lung cancer includes non-small cell lung carcinoma, small cell lung carcinoma. The non-small cell lung carcinoma may be lung adenocarcinoma or lung squamous cell carcinoma.

### Method for Predicting Prognosis of Cancer Patient

In one embodiment, the present invention provides a method for predicting prognosis of a cancer patient, including: measuring an expression level of an immunosuppressive receptor LILRB4 or an immunosuppressive receptor LILRB4 gene in a biological sample of the cancer patient, where a higher expression level of the immunosuppressive receptor LILRB4 or the immunosuppressive receptor LILRB4 gene in tumor infiltrating immune system cells in the biological sample than an expression level of the immunosuppressive receptor LILRB4 or the immunosuppressive receptor LILRB4 gene in a control indicates poor prognosis of the cancer patient.

The method of the present embodiment can be used to predict prognosis of a cancer patient. That is, it can be said that the method of the present embodiment is a method for predicting prognosis of a cancer patient. In the method of the present embodiment, poor prognosis means a lower survival rate, a high cancer recurrence rate, or the like. Conversely, in the method of the present embodiment, good prognosis means a higher survival rate, a low cancer recurrence rate, or the like. That is, the present invention encompasses a method for predicting prognosis of a cancer patient, including: measuring an expression level of an immunosuppressive receptor LILRB4 or an immunosuppressive receptor LILRB4 gene in a biological sample of the cancer patient, where an equivalent or lower expression level of the immunosuppressive receptor LILRB4 or the immunosuppressive receptor LILRB4 gene in tumor infiltrating immune system cells in the biological sample to/than an expression level of the immunosuppressive receptor LILRB4 or the immunosuppressive receptor LILRB4 gene in a control indicates good prognosis of the cancer patient.

In addition, the method of the present embodiment can be used for selecting patients to be applied with cancer treatment with a substance that inhibits binding between LILRB4 and fibronectin. That is, in the method of the present embodiment, it can be judged that the application of a substance that inhibits binding between LILRB4 and fibronectin is effective for the cancer treatment of patients who have been indicated to have high expression of LILRB4 or LILRB4 genes and poor prognosis. The substance that inhibits the binding between the fibronectin and the immunosuppressive receptor LILRB4 is not particularly limited as long as it is a substance having an activity of inhibiting the binding between the fibronectin and LILRB4, and examples thereof include anti-fibronectin antibodies, anti-LILRB4 antibodies, and fibronectin analogs, which will be described later.

In addition, the method of the present embodiment can be used for predicting an effect of a cancer therapeutic drug in a cancer patient. That is, in the method of the present embodiment, it can be predicted that the application, as a cancer therapeutic drug, of a substance that inhibits binding between LILRB4 and fibronectin will have an effect on cancer patients who have been indicated to have high expression of LILRB4 or LILRB4 genes and poor prognosis. Therefore, the method of the present embodiment can be used as a method for predicting an effect, as a cancer therapeutic drug in a cancer patient, of a substance that inhibits binding between fibronectin and an immunosuppressive receptor LILRB4, which will be described later.

In the method for predicting prognosis of a cancer patient according to the present embodiment, the target cancer patient may be a cancer patient who has been administered a cancer therapeutic drug. That is, in the method for predicting prognosis of a cancer patient according to the present embodiment, when the expression level of the immunosuppressive receptor LILRB4 or the immunosuppressive receptor LILRB4 gene in the tumor infiltrating immune system cells in the biological sample that has been administered the cancer therapeutic drug is lower than that in the biological sample of the cancer patient before the administration of the cancer therapeutic drug, the patient who has been administered the cancer therapeutic drug can be predicted to have good prognosis. Therefore, the method for predicting prognosis of a cancer patient according to the present invention can be used for evaluating the effect of a cancer therapeutic drug in a cancer patient who has been administered the cancer therapeutic drug. The cancer therapeutic drug is not particularly limited as long as it is a therapeutic drug for cancer, and examples thereof include immune checkpoint inhibitors, chemotherapeutic agents, and molecular target drugs. Examples of the immune checkpoint inhibitors include anti-PD-1 inhibitory antibodies, anti-PD-L1 inhibitory antibodies, and substances that inhibit binding between fibronectin and the immunosuppressive receptor LILRB4. The substances that inhibit binding between fibronectin and the immunosuppressive receptor LILRB4 are preferred because they exhibit therapeutic effects on patients with high expression levels of the immunosuppressive receptor LILRB4 by inhibiting the binding between fibronectin and the immunosuppressive receptor LILRB4. The substance that inhibits the binding between the fibronectin and the immunosuppressive receptor LILRB4 is not particularly limited as long as it is a substance having an activity of inhibiting the binding between the fibronectin and LILRB4, and examples thereof include anti-fibronectin antibodies, anti-LILRB4 antibodies, and fibronectin analogs, which will be described later.

The cancer targeted by the method of the present embodiment is not particularly limited, and examples thereof include solid cancer, more specifically, lung cancer, breast cancer, and liver cancer. The method is preferably used for lung cancer. The lung cancer includes non-small cell lung carcinoma, small cell lung carcinoma. The non-small cell lung carcinoma may be lung adenocarcinoma or lung squamous cell carcinoma.

In the method of the present embodiment, examples of the biological sample of the cancer patient include a tumor analyte collected by biopsy or surgery, and blood such as serum, plasma, or whole blood, lymph, tissue fluid, spinal fluid, body cavity fluid, digestive fluid, nasal fluid, lacrimal fluid, sweat, and urine of the cancer patient. The biological sample may be a biological sample itself collected from a cancer patient, or may be a biological sample obtained by subjecting a collected biological sample to a treatment such as dilution or concentration which is usually performed. The biological sample may be collected or prepared at the time of carrying out the measurement method, or may be collected or prepared and stored in advance.

The measuring of the expression level of LILRB4 in a biological sample can be carried out by an immunochemical measurement method using a specific binding substance for LILRB4 such as ELISA, Western blotting, immunohistochemical staining, cellular analysis using flow cytometry, or the like. The measuring of the expression level of LILRB4 genes can be carried out by microarray, RT-PCR, quantitative RT-PCR, or the like.

### Specific binding substance

Specific binding substances for LILRB4 include, for example, antibodies, fragments of antibodies, and aptamers. Antibodies that specifically bind to LILRB4 (hereinafter also referred to as anti-LILRB4 antibodies) can be fabricated, for example, by immunizing animals such as mice with LILRB4 or a fragment thereof as an antigen. Alternatively, they can be fabricated, for example, by screening a phage library.

The anti-LILRB4 antibodies may be either monoclonal antibodies or polyclonal antibodies as long as they bind to LILRB4, but monoclonal antibodies are preferably used.

The anti-LILRB4 monoclonal antibodies bind specifically to LILRB4. The anti-LILRB4 monoclonal antibodies may be any monoclonal antibodies that specifically bind to LILRB4, and may be antibodies that inhibit or do not inhibit binding between LILRB4 and its ligand, i.e., fibronectin. When patients to be applied with cancer treatment with substances that inhibit binding between LILRB4 and fibronectin are selected using the method for predicting prognosis of a cancer patient according to the present invention, antibodies that inhibit binding between LILRB4 and fibronectin are preferred. This is because, when the antibodies that inhibit binding between LILRB4 and fibronectin are used, the expression level of LILRB4 reflects the expression level of LILRB4 to which fibronectin binds, and thus patients to be applied with the substances that inhibit binding between LILRB4 and fibronectin can be selected more accurately.

Human LILRB4 containing a signal sequence contains an amino acid sequence represented by SEQ ID NO: 1. The human LILRB4 signal sequence is from methionine at position 1 to alanine at position 23 in SEQ ID NO: 1, and mature human LILRB4 contains an amino acid sequence from glycine at position 24 to tryptophan at position 258 in the amino acid sequence represented by SEQ ID NO: 1. Fibronectin, which is a ligand of LILRB4, can bind to human LILRB4 via an amino acid sequence represented by SEQ ID NO: 2 in the fibronectin. That is, the amino acid sequence represented by SEQ ID NO: 2 is a target sequence for human LILRB4 in the fibronectin.

Monoclonal antibodies can be fabricated according to known methods for fabricating monoclonal antibodies, for example, "Monoclonal Antibody" written by Hideaki Nagamune and Hiroshi Terada, Hirokawa Publishing Company (1990) and "Monoclonal Antibody" written by Jame W. Golding, 3rd edition, Academic Press, 1996. Monoclonal antibodies can also be fabricated by a DNA immunization method, and can be fabricated with reference to Nature 1992 Mar 12, 356, 152 to 154 and J. Immunol Methods Mar 1, 249, 147 to 154.

LILRB4 proteins or partial fragments (peptides) thereof, or vectors incorporating cDNAs encoding LILRB4 proteins can be used as antigens used in the fabrication of the anti-LILRB4 antibodies. In order to obtain monoclonal antibodies that recognize the higher-order structure of LILRB4, full-length LILRB4 vectors, which are constructs containing full-length LILRB4 genes, are the most suitable antigenic genes for immunization, but, in addition, constructs into which a partial region of the LILRB4 sequence is inserted can also be used as antigenic genes for immunization. The partial region of the human LILRB4 sequence is preferably a LILRB4 region (fibronectin binding site) in which the amino acid sequence represented by SEQ ID NO: 2, which is a target sequence for human LILRB4 in the fibronectin, binds to human LILRB4. The DNA immunization method can be carried out by subcutaneously injecting the above-described gene constructs alone or in combination into an animal (mouse, rat, or the like) using any of various gene transfer methods (for example, intramuscular injection, electroporation, gene gun, or the like) and incorporating the gene constructs into cells.

The antibody fragment is preferably an antibody fragment containing an antigen binding portion, and examples thereof can include F(ab')₂, F(ab)₂, Fab', Fab, Fv, scFv, mutants thereof, and fusion proteins and fusion peptides containing an antibody portion. The antibody fragment can be produced according to a known method for producing an antibody fragment.

As used herein, the sentence that antibodies "specifically bind" means that antibodies bind to LILRB4 without substantially binding to other polypeptides that are different from LILRB4.

Examples of the anti-LILRB4 monoclonal antibodies include anti-LILRB4 monoclonal antibodies in which an amino acid sequence of a complementarity determining region (hereinafter, also referred to as CDR) 1 of a heavy chain variable region (hereinafter, also referred to as VH) contains an amino acid sequence represented by SEQ ID NO: 3, an amino acid sequence of a CDR2 of the VH contains an amino acid sequence represented by SEQ ID NO: 4, an amino acid sequence of a CDR3 of the VH contains an amino acid sequence represented by SEQ ID NO: 5, an amino acid sequence of a CDR1 of a light chain variable region (hereinafter, also referred to as VL) contains an amino acid sequence represented by SEQ ID NO: 6, an amino acid sequence of a CDR2 of the VL contains an amino acid sequence represented by SEQ ID NO: 7, and an amino acid sequence of a CDR3 of the VL contains an amino acid sequence represented by SEQ ID NO: 8, and anti-LILRB4 monoclonal antibodies in which an amino acid sequence of the VH contains an amino acid sequence represented by SEQ ID NO: 9 and an amino acid sequence of the VL contains an amino acid sequence represented by SEQ ID NO: 10.

If necessary, the anti-LILRB4 monoclonal antibodies can be further purified before use. Techniques for purifying and isolating the anti-LILRB4 monoclonal antibodies include known methods, for example, salting out such as ammonium sulphate precipitation, gel filtration methods using Sephadex or the like, ion-exchange chromatography methods, and affinity purification methods using protein A column or the like.

An aptamer is a substance having the ability to specifically bind to a target substance. Examples of the aptamer include a nucleic acid aptamer and a peptide aptamer. A nucleic acid aptamer having the ability to specifically bind to a target peptide can be sorted by, for example, the systematic evolution of ligand by exponential enrichment (SELEX) method or the like. A peptide aptamer having the ability to specifically bind to a target peptide can be sorted by, for example, the two-hybrid method using yeast.

### Method for measuring expression level of LILRB4 or LILRB4 gene

The expression level of LILRB4 can be measured by an immunological measurement method using the above-described specific binding substance for LILRB4, such as ELISA, Western blotting, or immunohistochemical staining. The expression level of LILRB4 genes can be measured by microarray, RT-PCR, quantitative RT-PCR, or the like.

Examples of the immunological measurement method using the anti-LILRB4 monoclonal antibodies include an immunological measurement method for LILRB4 in a biological sample including: reacting LILRB4 in a biological sample with the anti-LILRB4 monoclonal antibodies or fragments thereof, adding labeled antibodies or fragments of the labeled antibodies in which labels bind to the anti-LILRB4 monoclonal antibodies or fragments of the antibodies to form immune complexes consisting of LILRB4, the anti-LILRB4 monoclonal antibodies or fragments of the antibodies, and the labeled antibodies or fragments of the labeled antibodies, and measuring an amount of labels in the formed immune complexes.

The immunological measurement methods are classified into enzyme immunoassay (EIA or ELISA), radioimmunoassay (RIA), fluorescent immunoassay (FIA), fluorescence polarization immunoassay (FPIA), chemiluminescent immunoassay (CLIA), electrochemiluminescent immunoassay, immunohistochemical staining and the like depending on the labels of the labeled detection antibodies, and any of them can be used in the immunological measurement methods. However, ELISA is preferable for a liquid phase sample and immunohistochemical staining is preferable for a solid sample such as a tissue section because the target for detection can be conveniently and rapidly measured.

After washing the immune complexes, LILRB4 in a biological sample can be measured by measuring the labels in the immune complexes. For example, in a case of ELISA, LILRB4 in a biological sample can be measured by reacting enzymes as labels with substrates of the enzymes and measuring absorbances of colored products (sandwich method). LILRB4 in a biological sample can also be measured by reacting anti-LILRB4 monoclonal antibodies or fragments of the antibodies immobilized on a solid support with LILRB4 in a biological sample, adding unlabeled anti-LILRB4 monoclonal antibodies or fragments of the antibodies (primary antibodies), further adding enzyme-labeled secondary antibodies to the unlabeled antibodies or fragments of the antibodies (secondary antibodies), and measuring the labels of the secondary antibodies. LILRB4 in a biological sample can also be measured by labeling the secondary antibodies with biotin, binding avidin or streptavidin labeled with enzymes or the like to biotin, labeling the secondary antibodies with enzymes or the like, and measuring the labels of the secondary antibodies.

LILRB4 in a biological sample can also be measured by adding unlabeled anti-LILRB4 monoclonal antibodies or fragments of the antibodies (primary antibodies) to LILRB4 or conjugates of LILRB4 and proteins such as BSA immobilized on a solid support to form immune complexes consisting of LILRB4 and the primary antibodies on the solid support, adding a biological sample, further adding secondary antibodies obtained by labeling antibodies against the unlabeled antibodies (secondary antibodies), and measuring the labels of the labeled secondary antibodies (competition method).

The solid support is not particularly limited as long as it can stably hold the antibodies or fragments of the antibodies. Preferred materials for the solid support include polymer materials such as polystyrene, polycarbonate, polyvinyltoluene, polypropylene, polyethylene, polyvinyl chloride, nylon, polymethacrylate, gelatin, agarose, cellulose, nitrocellulose, cellulose acetate, cellulose acetate, and polyethylene terephthalate; glass; ceramics; magnetic particles; and metals. Preferred forms of the solid support include tubes, beads, plates, microparticles such as latex, sticks and the like.

Examples of the labels include enzymes such as peroxidase and alkaline phosphatase in ELISA, radioactive substances such as ¹²⁵I, ¹³¹I, ³⁵S and ³H in RIA, fluorescent substances such as fluorescein isothiocyanate, rhodamine, dansyl chloride, phycoerythrin, tetramethylrhodamine isothiocyanate and near-infrared fluorescent materials in FPIA, enzymes such as luciferase and β-galactosidase, luminescent substrates which are converted into luminescent substances by the enzymes, and luminescent substances such as luciferin and aequorin in CLIA. In addition, nanoparticles such as gold colloids and quantum dots can be used as the labels.

In ELISA, when the enzyme is peroxidase, 3,3'-diaminobenzidine (DAB), 3,3', 5,5'-tetramethylbenzidine (TMB), O-phenylenediamine (OPD) or the like can be used as a substrate of the enzyme serving as the label, and, when the enzyme is alkaline phosphatase, p-nitropheny phosphate (pNPP) or the like can be used.

The measuring of the expression level of LILRB4 genes in a biological sample can be carried out by RT-PCR using a primer set capable of amplifying the LILRB4 genes, quantitative RT-PCR, or the like. Alternatively, the expression level of the LILRB4 genes may be measured by DNA-microarray analysis using a probe that specifically hybridizes with mRNAs of the LILRB4 genes, northern blotting, or the like.

### Primer set

The primer set capable of amplifying the LILRB4 genes is not particularly limited as long as it can amplify mRNAs of the LILRB4 genes by RT-PCR or the like.

The mRNAs of the LILRB4 genes have the base sequences described in the entries of the RefSeq database identified by the IDs described above. Since splicing variants and the like may be present for the mRNAs, the mRNAs of the LILRB4 genes are not limited to those identified by the RefSeq IDs described above.

For example, RT-PCR or the like is performed on a biological sample of a cancer patient using the primer set, and thus the mRNAs of the LILRB4 genes in the biological sample can be detected.

### Probe

The probe is not particularly limited as long as it can specifically hybridize with the mRNAs of the LILRB4 genes. The probe may be immobilized on a carrier to form a DNA microarray or the like. For example, the prognosis of a cancer patient can be predicted by contacting mRNAs extracted from tissue of a cancer patient with the DNA microarray and detecting the mRNAs of the LILRB4 genes hybridized with the probe.

In the method of the present embodiment, a higher expression level of LILRB4 or LILRB4 genes in tumor infiltrating immune system cells in a biological sample than an expression level of the LILRB4 or LILRB4 genes in a control indicates poor prognosis of the cancer patient. That is, it can be determined that the prognosis of the cancer patient is poor when the expression level of LILRB4 or LILRB4 genes in tumor infiltrating immune system cells in a biological sample is higher than the expression level of the LILRB4 or LILRB4 genes in a control. In the method of the present embodiment, an equivalent or lower expression level of LILRB4 or LILRB4 genes in tumor infiltrating immune system cells in a biological sample of a cancer patient to/than an expression level of the LILRB4 or LILRB4 genes in a control indicates good prognosis of the cancer patient. That is, it can be determined that the prognosis of the cancer patient is good when the expression level of LILRB4 or LILRB4 genes in tumor infiltrating immune system cells in a biological sample of the cancer patient is equivalent to or lower than the expression level of the LILRB4 or LILRB4 genes in a control.

Examples of the control include biological samples derived from a healthy person, for example, blood such as serum, plasma, or whole blood, lymph, tissue fluid, spinal fluid, body cavity fluid, digestive fluid, nasal fluid, lacrimal fluid, sweat, and urine derived from a healthy person, and normal tissues derived from a cancer patient. A higher expression level of the immunosuppressive receptor LILRB4 or immunosuppressive receptor LILRB4 genes in the tumor infiltrating immune system cells in the biological sample of the cancer patient than the expression level of the LILRB4 or LILRB4 genes in the control indicates poor prognosis of the cancer patient, and an equivalent or lower expression level thereof to/than the expression level of the LILRB4 or LILRB4 genes in the control indicates good prognosis of the cancer patient. Alternatively, a reference value may be set in advance based on the expression level of the LILRB4 or LILRB4 genes in the control, and a higher expression level of the LILRB4 or LILRB4 genes than the reference value may indicate poor prognosis of the cancer patient, and an equivalent or lower expression level thereof to/than the reference value may indicate good prognosis of the cancer patient. The control may be a mean value of the expression levels of LILRB4 or LILRB4 genes in TIL regions of a plurality of cancer patients. For example, the mean value of the expression levels of LILRB4 or LILRB4 genes in TIL regions of a plurality of cancer patients may be determined in advance and set as a reference value. A higher expression level of LILRB4 or LILRB4 genes than the reference value may indicate poor prognosis of the cancer patient, and an equivalent or lower expression level to/than the reference value may indicate good prognosis of the cancer patient.

The tumor infiltrating immune system cells are immune system cells found in tumors and include tumor infiltrating lymphocytes (TILs), myeloid-derived suppressor cells (MDSCs), tumor-associated macrophages (TAMs), tumor-associated neutrophils (TANs), tumor infiltrating dendritic cells, and the like. The TILs include intratumoral tumor infiltrating lymphocytes (intratumoral TILs) and stromal tumor infiltrating lymphocytes (stromal TILs). As used herein, the TIL region refers to a tumor stroma in which tumor infiltrating immune system cells including TILs are found. The TIL region in a biological sample can be qualitatively and quantitatively evaluated by microscopic examination of a pathological tissue specimen (a hematoxylin-eosin (HE) stained specimen, an immunohistochemically stained specimen, or the like) of a tumor analyte, cell analysis by flow cytometry, or the like. The TIL region is preferably a stromal region which is not a tumor region, i.e., a region where mononuclear cell infiltration is observed. More preferably, the TIL region is other than a region rich in stroma-associated granulocytes, a region of tertiary lymphoid structures, i.e., follicular or distal lymphocyte aggregates with germinal centers, a region with necrosis, a boundary of a tumor, i.e., outside of a para-tumor stromal region, a TIL region found in normal lung tissue, etc.

With respect to non-small cell lung carcinoma (NSCLC; adenocarcinoma and squamous cell carcinoma as targets for excision occupy most of them), it has been reported that the prognosis is better as the number of stromal TILs is larger.

As described above, it has been revealed that the number of immune cells in a local tumor site reflects the therapeutic reactivity and prognosis of a cancer-bearing patient. As will be described in the Examples below, even if there are many TILs in a biological sample of a cancer patient, the prognosis of the cancer patient is poor when the expression level of LILRB4 or LILRB4 genes on tumor infiltrating immune system cells including TILs is high. That is, even if the number of TILs in a biological sample of a cancer patient is large, the prognosis of the cancer patient can be determined to be poor when the expression level of LILRB4 or LILRB4 genes on tumor infiltrating immune system cells including TILs is high.

### Method for Predicting Effect of Cancer Therapeutic Drug

In one embodiment, the present invention provides a method for predicting an effect, as a cancer therapeutic drug in a cancer patient, of a substance that inhibits binding between fibronectin and an immunosuppressive receptor LILRB4. The method includes: measuring an expression level of an immunosuppressive receptor LILRB4 or an immunosuppressive receptor LILRB4 gene in a biological sample of the cancer patient. A higher expression level of the immunosuppressive receptor LILRB4 or immunosuppressive receptor LILRB4 gene in tumor infiltrating immune system cells in the biological sample than an expression level of the immunosuppressive receptor LILRB4 or the immunosuppressive receptor LILRB4 gene in a control indicates that the cancer therapeutic drug has an effect on the cancer patient.

The method of the present embodiment can be used to predict an effect of a substance that inhibits binding between fibronectin and an immunosuppressive receptor LILRB4 as a cancer therapeutic drug in a cancer patient. That is, the method of the present embodiment can be said to be a method for predicting an effect of a substance that inhibits binding between fibronectin and an immunosuppressive receptor LILRB4 as a cancer therapeutic drug in a cancer patient. In the method of the present embodiment, poor prognosis means a lower survival rate, a high cancer recurrence rate, or the like. The cancer targeted by the method of the present embodiment is not particularly limited, and examples thereof include solid cancer, more specifically, lung cancer, breast cancer, and liver cancer. The method is preferably used for lung cancer. The lung cancer includes non-small cell lung carcinoma, small cell lung carcinoma. The non-small cell lung carcinoma may be lung adenocarcinoma or lung squamous cell carcinoma.

In the method of the present embodiment, the biological sample of the cancer patient and the control are the same as those used in the method for predicting prognosis of a cancer patient, and the measuring of the expression level of LILRB4 in the biological sample of the cancer patient and the measuring of the expression level of LILRB4 genes in the biological sample of the cancer patient are the same as those of the method for predicting prognosis of a cancer patient.

In the method of the present embodiment, a higher expression level of LILRB4 or LILRB4 genes in tumor infiltrating immune system cells in a biological sample of a cancer patient is than the expression level of the LILRB4 or LILRB4 genes in a control indicates that the substance that inhibits binding between fibronectin and the immunosuppressive receptor LILRB4 as a cancer therapeutic drug to be administered to the cancer patient is effective for the cancer patient. That is, when the expression level of LILRB4 or LILRB4 genes in tumor infiltrating immune system cells in a biological sample of a cancer patient is higher than the expression level of the LILRB4 or LILRB4 genes in a control, it can be predicted that the cancer therapeutic drug has an effect on the cancer patient.

In the method of the present embodiment, the substance that inhibits the binding between the fibronectin and the immunosuppressive receptor LILRB4 is not particularly limited as long as it is a substance having an activity of inhibiting the binding between the fibronectin and LILRB4, and examples thereof include anti-fibronectin antibodies, anti-LILRB4 antibodies, and fibronectin analogs.

Examples of the anti-LILRB4 antibodies include anti-human LILRB4 monoclonal antibodies in which an amino acid sequence of a complementarity determining region (hereinafter, also referred to as CDR) 1 of a heavy chain variable region (hereinafter, also referred to as VH) contains an amino acid sequence represented by SEQ ID NO: 3, an amino acid sequence of a CDR2 of the VH contains an amino acid sequence represented by SEQ ID NO: 4, an amino acid sequence of a CDR3 of the VH contains an amino acid sequence represented by SEQ ID NO: 5, an amino acid sequence of a CDR1 of a light chain variable region (hereinafter, also referred to as VL) contains an amino acid sequence represented by SEQ ID NO: 6, an amino acid sequence of a CDR2 of the VL contains an amino acid sequence represented by SEQ ID NO: 7, and an amino acid sequence of a CDR3 of the VL contains an amino acid sequence represented by SEQ ID NO: 8, and anti-human LILRB4 monoclonal antibodies in which an amino acid sequence of the VH contains an amino acid sequence represented by SEQ ID NO: 9 and an amino acid sequence of the VL contains an amino acid sequence represented by SEQ ID NO: 10.

In the method for predicting an effect of a cancer therapeutic drug according to the present embodiment, the fibronectin analog includes any fibronectin analogs as long as they have an action of inhibiting binding between fibronectin and LILRB4, and is, for example, any one of the following peptides (a) to (c):
(a) a peptide containing an amino acid sequence represented by SEQ ID NO: 2;
(b) a peptide containing an amino acid sequence in which one to several amino acids are deleted, inserted, substituted, or added in the amino acid sequence represented by SEQ ID NO: 2, and having the ability to bind to a fibronectin binding site of an immunosuppressive receptor LILRB4; and
(c) a peptide containing an amino acid sequence having 80% or greater identity with the amino acid sequence represented by SEQ ID NO: 2, and having the ability to bind to a fibronectin binding site of an immunosuppressive receptor LILRB4.

The amino acid sequence identity is 80% or greater, preferably 85% or greater, more preferably 90% or greater, even more preferably 95% or greater, and still even more preferably 98% or greater. The number of deletions, substitutions, or additions in the amino acid sequence is preferably 1 to 5, more preferably 1 to 4, even more preferably 1 to 3, and still even more preferably 1 to 2. The amino acid sequence identity can be determined by a BLAST search provided on the GenBank database.

The fibronectin analog may be a fibronectin analog in which any one of the peptides (a) to (c) is fused with an Fc region of immunoglobulin G.

### Kit for Predicting Prognosis of Cancer Patient

In one embodiment, the present invention provides a kit for predicting prognosis of a cancer patient, including a reagent for measuring an expression level of a biomarker for predicting prognosis of a cancer patient consisting of an immunosuppressive receptor LILRB4 or immunosuppressive receptor LILRB4 gene. The kit of the present embodiment can be used in the method for predicting prognosis of a cancer patient according to the present invention.

The kit of the present embodiment can be used to predict the prognosis of a cancer patient. In addition, it is possible to obtain an appropriate selection index for a cancer treatment strategy. The kit for predicting prognosis of a cancer patient according to the present embodiment can also be said to be a diagnostic drug for predicting prognosis of cancer. The cancer targeted by the kit for predicting prognosis of a cancer patient of the present embodiment is not particularly limited, and examples thereof include solid cancer, more specifically, lung cancer, breast cancer, and liver cancer. The kit is preferably used for lung cancer. The lung cancer includes non-small cell lung carcinoma, small cell lung carcinoma. The non-small cell lung carcinoma may be lung adenocarcinoma or lung squamous cell carcinoma.

### Reagent for measuring expression level of biomarker

Examples of the reagent for measuring an expression level of a biomarker for predicting prognosis of a cancer patient consisting of an immunosuppressive receptor LILRB4 or immunosuppressive receptor LILRB4 gene include the above-described specific binding substances for LILRB4, primer sets capable of amplifying LILRB4 genes, and probes that specifically hybridize with mRNAs of LILRB4 genes. The specific binding substances for LILRB4 include the above-described anti-LILRB4 antibodies or fragments of the antibodies, aptamers and the like. The primer sets capable of amplifying LILRB4 genes and probes that specifically hybridize with mRNAs of LILRB4 genes include the primer sets and probes described above.

The kit of the present embodiment may further include known reagents for immunohistochemical staining, reagents for ELISA, Western blotting, or RT-PCR, such as labeling reagents, buffers, chromogenic substrates, secondary antibodies, blocking agents, instruments necessary for measurement, controls, and reference documents indicating reference values of expression levels of LILRB4 or LILRB4 genes of controls.

Since the kit for predicting prognosis of a cancer patient according to the present embodiment can also be used to predict prognosis of a cancer patient who has been administered a cancer therapeutic drug, the kit of the present embodiment can also be used in a method for evaluating an effect of a cancer therapeutic drug in a patient who has been administered a cancer therapeutic drug. For example, the prognosis of a cancer patient is predicted using the kit of the present embodiment; the cancer patient predicted to have poor prognosis is treated by administering a cancer therapeutic drug; the prognosis of the cancer patient is predicted again using the kit of the present embodiment after the treatment; and if the prognosis is indicated to be good, the cancer therapeutic drug administered to the cancer patient can be evaluated as effective. In addition, the kit for predicting prognosis of a cancer patient according to the present embodiment can also be used in the method for predicting an effect, as a cancer therapeutic drug according to the present invention, of a substance that inhibits binding between fibronectin and LILRB4. For example, the prognosis of a cancer patient is predicted using the kit of the present embodiment; and to a cancer patient predicted to have poor prognosis, the application of a substance having an activity of inhibiting binding between fibronectin and LILRB4 as a cancer therapeutic drug can be predicted to have a cancer therapeutic effect.

### EXAMPLES

Next, the present invention will be described in more detail by way of examples, but is not limited to the following examples.

### [Experimental Example 1] Evaluation of Prognosis of Non-Small Cell Lung Carcinoma Patient

For tissue samples of 40 cases of non-small cell lung carcinoma patients (age mean value: 68 years, 35% female), for example, the SUVmax, the presence or absence of EGFR mutation, the degree of differentiation, the presence or absence of lymph node metastasis, vascular invasion, lymphatic invasion, and pleural invasion, and the like, which are considered to be pathological prognostic factors, were examined. The results are indicated in Table 1.

**[Table 1]**

| Clinicopathological features | | Total number of n [proportion (%)] |
|---|---|---|
| Number of patients | | 40 |
| Age [mean (SD)] | | 68.5 (9.30) |
| Number of females | | 14 (35.0%) |
| pTNM classification | IA | 21 (52.5%) |
| | IB | 10 (25.0%) |
| | IIA | 5 (12.5%) |
| | IIB | 3 (7.5%) |
| Treatment | Bilobectomy | 2 (5.0%) |
| | Lobectomy | 31 (77.5%) |
| | Segmentectomy | 3 (7.5%) |
| | Partial resection | 4 (10.0%) |
| Mean tumor diameter [mm; mean (SD)] | | 30.28 (19.0) |
| Suv max [mean (SD)] | | 4.7 (4.39) |
| EGFR mutation | + | 12 (31.6%) |
| | - | 26 (68.4%) |
| Tissue pathology | Adenocarcinoma | 23 (57.5%) |
| | Squamous cell carcinoma | 17 (42.5%) |
| Degree of differentiation | G1 | 16 (42.1%) |
| | G2 | 16 (42.1%) |
| | G3 | 6 (15.8%) |
| | G4 | 0 (0%) |
| Lymph node metastasis | + | 5 (12.5%) |
| | - | 35 (87.5%) |
| Vascular invasion | + | 17 (42.5%) |
| | - | 23 (57.5%) |
| Lymphatic invasion | + | 8 (20.0%) |
| | - | 32 (80.0%) |
| Pleural invasion | + | 13 (32.5%) |
| | - | 27 (67.5%) |

The tissue samples used in the present experimental example were resected examples, and thus often had early lung cancer. Therefore, all cases were in stage I or stage II, as shown in Table 1. Also, the mean tumor diameter was 30 mm.

Cancer cells are active in glucose metabolism, and thus show an increased uptake when administered ¹⁸F-fluorodeoxyglucose; FDG) which is a radio-labeled pseudo-glucose. The FDG-PET examination is an examination in which diagnosis is performed based on an imaged distribution of FDG in the body. In the FDG-PET examination, the maximum value after standardization with the patient volume is SUVmax. There are many reports that the threshold of SUVmax to differentiate between benign and malignant is about 2. As shown in Table 1, the SUVmax was 4.7 for the tissue samples in the present experimental example.

EGFR (Epidermal Growth Factor Receptor) is one of the proteins involved in cell proliferation, and when a mutation occurs in the EGFR gene, abnormal EGFR is presumed to be produced, which causes cell proliferation even when not needed, so that cancer tends to occur. In general, it is known that an increase in EGFR mutation results in poor prognosis of a cancer patient. As shown in Table 1, EGFR mutation was observed in 31.6% of the total of the tissue samples in the present experimental example.

The degree of differentiation is an index indicating how much the morphology of a cell (alveolar epithelial cell) that is the origin of cancer is maintained. The degree of differentiation decreases from G1 to G3, and G4 represents undifferentiated. Highly differentiated cancer has a morphology similar to that of alveolar epithelial cells, but, as the degree of differentiation decreases, heteromorphism increases, and, in general, the malignancy also increases. As shown in Table 1, of the biological samples in the present experimental example, 42.1% had G1, 42.1% had G2, 15.8% had G3, and 0% had G4.

Vascular invasion and lymphatic invasion indicate whether tumor infiltration is present in minute blood vessels or lymphatic vessels in tumor tissue, and it is known that the presence of tumor infiltration causes poor prognosis even at the same disease stage. Pleural invasion indicates whether invasion is present in the pleura at the lung surface, and it is known that, since the pleura is rich in lymphocytes, the presence of pleural invasion causes poor prognosis of cancer even at the same disease stage. As shown in Table 1, in the present experimental example, the patients with vascular invasion accounted for 42.5% of the total, the patients with lymphatic invasion accounted for 20.0% of the total, and the patients with pleural invasion accounted for 32.5% of the total.

### [Experimental Example 2] Production of Recombinant Human LILRB4

A cDNA of an extracellular domain (from glycine at position 24 to glycine at position 219; hereinafter also referred to as exB4) of human LILRB4 obtained by substituting isoleucine at position 156 with cysteine and substituting histidine at position 166 with cysteine in human LILRB4 having an amino acid sequence represented by SEQ ID NO: 1, which is a codon optimized for expression in E. coli, was chemically synthesized and inserted into a pUCIDT KAN vector (available from Integrated DNA Technologies) to construct a plasmid pUCIDT-KAN/exB4 (I156C/H166C).

This plasmid was digested with NdeI and XhoI, and the resulting LILRB4cDNA fragment was ligated to the NdeI/XhoI site of pET-26M in which a portion between NdeI and NcoI of pET-26b (+) (available from Novagen) was modified to Gly-× 6 His tag-Ala. The resulting plasmid pET-26M/exB4 (I156C/H166C) was transfected into E. coli BL21 (DE3), and expression of LILRB4 proteins was induced by addition of isopropyl-β-D-thiogalactopyranoside (IPTG) to a medium. After E. coli was cultured, the bacterial cells were suspended in a lysis buffer (50 mM Tris-HCl, 300 mM NaCl, pH: 8.0), sonicated, and centrifuged to collect inclusion bodies containing exB4. The resulting inclusion bodies were solubilized in a denaturing buffer (6M guanidine-HCl, 50mM Tris-HCl, 100mM NaCl, 10mM EDTA, 10mM DTT, pH: 8.0). To refold exB4, the denatured exB4 solutions were added dropwise to a refolding buffer (100mM Tris-HCl, 2mM EDTA, 0.4M L-arginine-HCl, 0.5mM oxidized glutathione) at 4°C. The refolded exB4 was dialyzed into PBS (-) and used as an immunogen.

### [Experimental Example 3] Fabrication of Anti-Human LILRB4 Monoclonal Antibody

Human LILRB4-specific monoclonal antibodies were fabricated by immunizing mice with exB4 obtained in Experimental Example 2 using the mouse iliac lymph node method (Y Sado et al., Acta Histochem. Cytochem., 39: 89-94, 2006). Next, human LILRB4-specific monoclonal antibody-producing hybridomas were selected by screening monoclonal antibody-containing culture supernatants by ELISAs using the following recombinant proteins. Next, the selected human LILRB4-specific monoclonal antibody-producing hybridomas were cultured, and anti-human LILRB4 monoclonal antibodies were obtained from the resulting culture solutions.

Recombinant human LILRB1 (#16014-H08H available from Sino Biological)
Recombinant human LILRB2 (#14132-H08H available from Sino Biological)
Recombinant human LILRB3 (#11978-J08H available from Sino Biological)
Recombinant human LILRB4 (#16742-H08H available from Sino Biological)
Recombinant human LILRB5 (#17221-H08H available from Sino Biological)
Recombinant human LILRA1 (#9226-T4-050 available from R & D Systems)
Recombinant human LILRA2 (#9040-T4-050 available from R & D Systems)
Recombinant human LILRA3 (#13549-H08H available from Sino Biological)
Recombinant human LILRA4 (#16058-H08H available from Sino Biological)
Recombinant human LILRA5 (#8956-T4-050 available from R & D Systems)
Recombinant human LILRA6 (#9088-T4-050 available from Sino Biological)
Recombinant human LILRB4 (#9095-T4-050 available from R & D Systems)

ELISA was performed by using F(ab')₂mouse anti-6 × His tag monoclonal antibodies (clones Nos. 21 to 48 available from ITM) as capture antibodies and HRP-conjugated rabbit anti-mouse IgG-Fc (available from ITM) as a detection antibody.

### [Experimental Example 4] Evaluation of LILRB4 Expression on TIL Region of Non-Small Cell Lung Carcinoma Patient (1)

Immunohistochemical staining was performed on the stromal TIL regions of the 40 cases of non-small cell lung carcinoma patients used in Experimental Example 1, using goat anti-LILRB4 polyclonal antibodies (available from R & D Systems), and expression levels of LILRB4 were evaluated in a double blind manner by two examiners. According to a method generally used for breast cancer, the numbers of LILRB4 positive cells were evaluated by being classified into the following positive cell scores of from 0 to 5 according to proportions of LILRB4 positive cell area to tumor stromal area.
0: less than 1%
1: 1% or greater and less than 5%
2: 5% or greater and less than 10%
3: 10% or greater and less than 20%
4: 20% or greater and less than 50%
5: 50% or greater

FIG. 1 shows representative examples of LILRB4 immunohistochemical staining in a case of lung adenocarcinoma. In No. 9605, LILRB4 positive cells were hardly observed in the stroma except for the tumor cells, and the number of LILRB4 positive cells was evaluated as "0". On the other hand, in No. 0577, the number of LILRB4 positive cells in the area of the stroma was determined to be 20% or greater and less than 50% and evaluated as "4".

In the 40 cases of cancer patient tissue samples used in Experimental Example 1, the median value for the evaluation of the immunohistochemical staining of the TIL region for LILRB4 was 2. Therefore, cases in which the proportion of LILRB4 positive cell area to tumor stromal area was less than 10% (positive cell score: from 0 to 2) were defined as a low expression group, and cases in which the proportion of LILRB4 positive cell area to tumor stromal area was 10% or greater (positive cell score: 3 or more) were defined as a high expression group. For each of the low expression group and the high expression group, the SUVmax, the presence or absence of EGFR mutation, the degree of differentiation, the presence or absence of lymph node metastasis, vascular invasion, lymphatic invasion, and pleural invasion, and the like, which are pathological prognostic factors, were examined in the same manner as in Experimental Example 1. The results are indicated in Table 2. Of the above 40 cases, 9 cases fell in the high expression group and 31 cases fell in the low expression group, and the number of patients and the proportion in each item are as shown in Table 2.

**[Table 2]**

| Clinicopathological features | Total number of n [proportion (%)] | LILRB4 expression | | p Value | | |
|---|---|---|---|---|---|---|
| | | High expression (3 or more) | Low expression (from 0 to 2) | | | |
| Number of patients | 40 | 9 | 31 | | | |
| Age [mean (SD)] | 68.5 (9.30) | 68.0 | 70.0 | 0.340 | Mann-Whitney test | |
| Number of females | | 14 (35.0%) | 2 | 12 | 0.453 | Mann-Whitney test |
| pTNM classification | IA | 21 (52.5%) | 4 (44.4%) | 17 (54.8%) | | |
| | IB | 10 (25.0%) | 3 (33.3%) | 7 (22.6%) | | |
| | IIA | 5 (12.5%) | 1 (11.1%) | 5 (16.1%) | | |
| | IIB | 3 (7.5%) | 1 (11.1%) | 2 (6.5%) | 0.853 | Chi-square |
| Treatment | Bilobectomy | 2 (5.0%) | 0 (0%) | 2 (6.5%) | | |
| | Lobectomy | 31 (77.5%) | 8 (88.9%) | 23 (74.2%) | | |
| | Segmentectomy | 3 (7.5%) | 0 (0%) | 3 (9.7%) | | |
| | Partial resection | 4 (10.0%) | 1 (11.1%) | 3 (9.7%) | 0.646 | Chi-square |
| Mean tumor diameter [mm; mean (SD)] | | 30.28 (19.0) | 36.3 | 28.5 | 0.065 | Mann-Whitney test |
| Suv max [mean (SD)] | | 4.7 (4.39) | 10.0 | 4.4 | 0.030 | Mann-Whitney test |
| EGFR mutation | + | 12 (31.6%) | 0 (0%) | 12 (40.0%) | | |
| | - | 26 (68.4%) | 8(100%) | 18 (60.0%) | 0.039 | Fisher's exact test* |
| Tissue pathology | Adenocarcinoma | 23 (57.5%) | 2 (22.2%) | 21 (67.7%) | | |
| | Squamous cell carcinoma | 17 (42.5%) | 7 (77.8%) | 10 (32.3%) | 0.023 | Fisher's exact test |
| Degree of differentiation | G1 | 16 (42.1%) | 1 (11.1%) | 15 (48.4%) | | |
| | G2 | 16 (42.1%) | 5 (55.6%) | 11 (35.5%) | | |
| | G3 | 6 (15.8%) | 3 (33.3%) | 3 (9.7%) | | |
| | G4 | 0 (0%) | 0 (0%) | 0 (0%) | 0.064 | Chi-square** |
| Lymph node metastasis | + | 5 (12.5%) | 1 (11.1%) | 4 (13.0%) | | |
| | - | 35 (87.5%) | 8 (88.9%) | 27 (87.1%) | 1.000 | Fisher's exact test |
| Vascular invasion | + | 17 (42.5%) | 7 (77.8%) | 10 (32.2%) | | |
| | - | 23 (57.5%) | 2 (22.2%) | 21 (67.7%) | 0.023 | Fisher's exact test |
| Lymphatic invasion | + | 8 (20.0%) | 1 (11.1%) | 7 (22.6%) | | |
| | - | 32 (80.0%) | 8 (88.9%) | 24 (77.4%) | 0.655 | Fisher's exact test |
| Pleural invasion | + | 13 (32.5%) | 4 (44.4%) | 9 (29.0%) | | |
| | - | 27 (67.5%) | 5 (55.6%) | 22 (70.1%) | 0.437 | Fisher's exact test |

| | | | | | | |
|---|---|---|---|---|---|---|
| *: Absence of EGFR mutation in 2 patients (n = 38) **: Absence of degree of differentiation in 2 patients (n = 38) | | | | | | |

As shown in Table 2, the SUVmax value in the FDG-PET test is significantly high in the high LILRB4 expression group, and it can be determined that the high LILRB4 expression group has high malignancy and poor prognosis of cancer. In addition, the number of patients positive for vascular invasion, which is a poor prognostic factor, was significantly large in the high LILRB4 expression group. Thus, this analysis presented a result that a plurality of poor prognostic factors was observed in the high LILRB4 expression group, although it is presumed that, since the analysis targeted early lung cancer, the prognosis was good as a whole and there was little difference between the two groups. From this result, it was revealed that the prognosis of cancer patients can be predicted by examining the expression of LILRB4 in tumor infiltrating immune system cells in the TIL region of the cancer patients.

Although a significant difference was observed between EGFR mutation and tissue type, EGFR mutation is observed in about 50% of Japanese lung adenocarcinoma patients, but is reported to be observed in 5% or less of Japanese squamous cell carcinoma patients. In this study, similar tendencies were observed for EGFR mutation and tissue type.

As shown in Table 2, the proportion of lung squamous cell carcinoma patients in the high LILRB4 expression group was as high as 77.8%. From this, it was revealed that the LILRB4 expression in the TIL region is high in lung squamous cell carcinoma.

### [Experimental Example 5] Analysis of Prognosis of Non-Small Cell Lung Carcinoma Patient

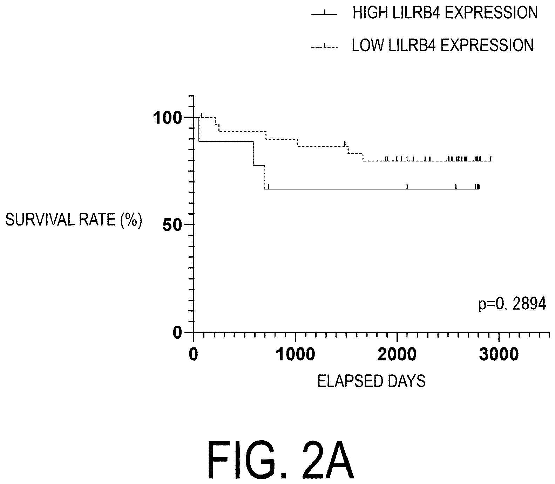

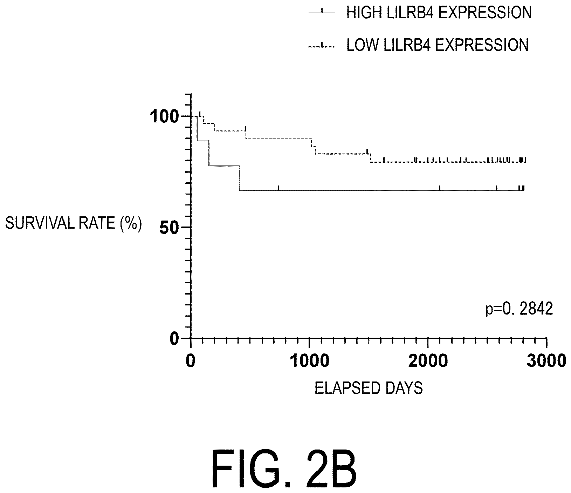

In the patients of Experimental Example 1, the overall survival rate (the proportion of the number of patients excluding dead patients to the total number of patients) and the recurrence-free survival rate (the proportion of the number of patients excluding dead patients and recurrent patients to the total number of patients) were examined for the high LILRB4 expression group and the low expression group. The overall survival rate is illustrated in FIG. 2A, and the recurrence-free survival rate is illustrated in FIG. 2B.

As shown in FIGS. 2A and 2B, no significant difference was observed in both the overall survival rate and the recurrence-free survival rate, but the low LILRB4 expression group was superior both in overall survival rate and in recurrence-free survival rate over the entire period. Also from this result, when LILRB4 is highly expressed in the TIL region, the prognosis of the cancer patients is presumed to be poor.

### [Experimental Example 6] Test on Inhibition of LILRB4-LILRB4 Ligand Binding by Anti-Human LILRB4 Monoclonal Antibody

The anti-human LILRB4 monoclonal antibodies obtained in Experimental Example 3 were examined for their ability to inhibit the binding of fibronectin to human LILRB4 using LILRB4 chimeric receptor GFP reporter cells.

Reporter cells derived from mouse T-cell hybridoma cells 2B4 express a chimeric receptor consisting of an extracellular domain of human LILRB4 fused to human activating paired immunoglobulin-like receptor β (PILRβ) and activating signal adaptor protein DAP12, and have green fluorescent protein (GFP) genes whose expression is induced under the control of nuclear transcription factors of activated T-cells (NFAT) (K Hirayasu et al., Nat. Microbiol. 1: 16054, 2016). The LILRB4 reporter cells were used to examine the inhibitory effect on binding between LILRB4 and fibronectin of the anti-LILRB4 monoclonal antibodies obtained in Experimental Example 3. Prior to the start of the test, recombinant proteins (hereinafter also referred to as FN30-Fc) in which the N-terminal (30 kDa) of human fibronectin (#9911 available from Sigma, hereinafter also referred to as FN30) was fused with Fc of mouse IgG2a were fabricated by the method described in WO2021/029318, and each well of a 96-well flat-bottom plate was precoated with 50 µL of PBS (-) containing 10 µg/mL FN30-Fc at room temperature.

Immediately before addition of the LILRB4 reporter cells, the wells were washed three times with PBS (-) for 1 hour. Some of the wells were kept uncoated for negative controls. The LILRB4 reporter cells were harvested from the cultures and placed on ice until the start of the test. In 50 µL of hybridoma culture supernatants containing anti-LILRB4 monoclonal antibodies, 5 × 10⁴ B4 reporter cells were suspended, and incubated on ice for 30 minutes with gentle mixing every 10 minutes. The LILRB4 reporter cells were then added directly to FN30-Fc coated or uncoated 96-well plates, without being washed. Next, 50 µL of a medium (RPMI-1640 supplemented with 10% FBS, 50 µM 2-mercaptoethanol, 0.1 U/ml penicillin, 0.1 µg/mL streptomycin) containing 60 µg/mL of recombinant human ApoE (#CI02 available from Novoprotein) was added to the LILRB4 reporter cells. As a control, 50 µL of a medium free of human ApoE was added to the LILRB4 reporter cells. The plate was then centrifuged at 400 × g for 3 minutes to allow the LILRB4 reporter cells to settle to the bottom of the plate. The LILRB4 reporter cells were incubated at 37°C for 24 hours, and the expression level of GFP proteins was measured by flow cytometry BDFACSAria^{™} III (available from BD Biosciences). The data was analyzed by FlowJo^{™} software (BD Biosciences). As a result, it was confirmed that the anti-human LILRB4 monoclonal antibody #10 has a good effect of inhibiting the binding of fibronectin and ApoE to human LILRB4.

Next, mRNA was extracted from the hybridoma strain producing the anti-human LILRB4 monoclonal antibody #10 by a routine method; the base sequences of VH and VL of the anti-human LILRB4 monoclonal antibody #10 were determined by the dideoxy method; and the amino acid sequences encoded by these base sequences were determined. Further, amino acid sequences corresponding to CDR regions were identified in the obtained amino acid sequences, and the base sequences and amino acid sequences of VH and VL were defined as CDR regions. As a result, it was determined that the amino acid sequence of VH of the anti-human LILRB4 monoclonal antibody #10 contains an amino acid sequence represented by SEQ ID NO: 9, and the amino acid sequence of VL contains an amino acid sequence represented by SEQ ID NO: 10. In addition, it was determined that the amino acid sequence of CDR 1 of VH of the anti-human LILRB4 monoclonal antibody #10 contains an amino acid sequence represented by SEQ ID NO: 3, the amino acid sequence of CDR2 of VH contains an amino acid sequence represented by SEQ ID NO: 4, the amino acid sequence of CDR3 of VH contains an amino acid sequence represented by SEQ ID NO: 5, the amino acid sequence of CDR1 of VL contains an amino acid sequence represented by SEQ ID NO: 6, the amino acid sequence of CDR2 of VL contains an amino acid sequence represented by SEQ ID NO: 7, and the amino acid sequence of CDR3 of VL contains an amino acid sequence represented by SEQ ID NO: 8.

### [Experimental Example 7] Evaluation of LILRB4 Expression in TIL Region of Lung Squamous Cell Carcinoma Patient

Immunohistochemical staining was performed using the anti-human LILRB4 monoclonal antibody #10 obtained in Experimental Example 6, which inhibits binding between fibronectin and human LILRB4, on each of the lung squamous cell carcinoma tissue specimens whose LILRB4 expression was previously determined to be strongly positive (High), medium positive (Medium) or weakly positive (Low), with goat anti-human LILRB4 polyclonal antibodies (available from R & D Systems). The results are illustrated in FIG. 3. As illustrated in FIG. 3, it was confirmed that LILRB4 was expressed in the TIL region of lung squamous cell carcinoma patients by using anti-human LILRB4 antibodies that inhibit binding between fibronectin and human LILRB4. Since the anti-LILRB4 antibodies that inhibit binding between fibronectin and human LILRB4 accurately reflect the expression level of LILRB4 to which the fibronectin binds in terms of its physiological function, this result indicates that the anti-LILRB4 antibodies that inhibit binding between fibronectin and human LILRB4 can be used to more accurately select patients for whom the anti-LILRB4 antibodies are effective than antibodies that do not inhibit binding between fibronectin and human LILRB4.

### [Experimental Example 8] Evaluation of LILRB4 Expression in TIL Region of Non-Small Cell Lung Carcinoma Patient (2)

The SUVmax, the presence or absence of EGFR mutation, the degree of differentiation, the presence or absence of lymph node metastasis, vascular invasion, lymphatic invasion, and pleural invasion, and the like were examined in the same manner as in Experimental Example 4 using 142 cases of lung adenocarcinoma patients and 97 cases of lung squamous cell carcinoma patients, i.e., 239 cases in total, using goat anti-LILRB4 polyclonal antibodies (available from R & D Systems). The results are shown in Table 3. Of the above 239 cases, 75 cases fell in the high expression group and 164 cases fell in the low expression group, and the number of patients and the proportion in each item are as shown in Table 3.

**Table 3**

| Clinicopathological features | Total number of n [proportion (%)] | LILRB4 expression | | p Value | | |
|---|---|---|---|---|---|---|
| | | High expression (3 or more) | Low expression (from 0 to 2) | | | |
| | | | | | | |
| Number of patients | | 239 | 75 (31.4%) | 164 (68.6%) | | |
| Age [mean (SD)] | | 70 (65-75) | 70 (65-76) | 70.5 (64-75) | 0.514 | Mann-Whitney test |
| Number of females | | 85 (35.6%) | 19 (25.3%) | 66 (40.2%) | 0.029 | Mann-Whitney test |
| Number of patients with smoking history (including current smoking) | | 171 (29.7%) | 65 (86.7%) | 106 (64.6%) | < 0.001 | Mann-Whitney test |
| pTNM classification | I | 168 (70.3%) | 52 (69.3%) | 116 (70.7%) | | |
| | II | 41 (17.2%) | 10 (13.3%) | 8 (4.9%) | | |
| | III | 28 (11.7%) | 12 (16.0%) | 16 (9.8%) | | |
| | IV | 2 (0.8%) | 1 (1.3%) | 1 (0.6%) | 0.373 | Chi-square |
| Mean tumor diameter [mm; mean (SD)] | | 24 (17-32) | 25 (17-35) | 23 (17-32) | 0.816 | Mann-Whitney test |
| Suv max [mean (SD)] | | 4.7 (2-8.38) | 6.8 (3.4-9.6) | 4.3 (1.8-7.6) | 0.003 | Mann-Whitney test* |
| EGFR mutation | + | 68 (32.1%) | 9 (13.6%) | 59 (40.4%) | | |
| | - | 144 (67.9%) | 57 (86.4%) | 87 (59.6%) | < 0.001 | Fisher's exact test** |
| Tissue pathology | Adenocarcinoma | 142 (59.4%) | 26 (34.7%) | 116 (70.7%) | | |
| | Squamous cell carcinoma | 97 (40.6%) | 49 (65.3%) | 48 (29.3%) | < 0.0001 | Fisher's exact test |
| Degree of differentiation | G1 | 101 (44.7%) | 15 (22.1%) | 86 (54.4%) | | |
| | G2 | 92 (40.7%) | 38 (55.9%) | 54 (34.2%) | | |
| | G3 | 33 (14.6%) | 15 (22.1%) | 18 (11.4%) | < 0.0001 | Chi-square*** |
| Lymph node metastasis | + | 44 (18.4%) | 17 (22.7%) | 27 (16.5%) | | |
| | - | 195 (81.6%) | 58 (77.3%) | 137 (83.5%) | 0.282 | Fisher's exact test |
| Vascular invasion | + | 101 (42.3%) | 39 (52.0%) | 62 (37.8%) | | |
| | - | 138 (57.7%) | 36 (48.0%) | 102 (62.2%) | 0.048 | Fisher's exact test |
| Lymphatic invasion | + | 73 (30.5%) | 21 (28.0%) | 52 (31.7%) | | |
| | - | 166 (69.5%) | 54 (72.0%) | 112 (68.3%) | 0.650 | Fisher's exact test |
| Pleural invasion | + | 66 (27.6%) | 23 (30.7%) | 43 (26.2%) | | |
| | - | 173 (72.4%) | 52 (69.3%) | 121 (73.8%) | 0.533 | Fisher's exact test |

| | | | | | | |
|---|---|---|---|---|---|---|
| *: Absence of SUV max in 7 patients (n = 232) **: Absence of EGFR mutation in 27 patients (n = 212) ***: Absence of degree of differentiation in 13 patients (n = 226) | | | | | | |

As shown in Table 3, the SUVmax value in the FDG-PET test is significantly high in the high LILRB4 expression group, and it can be determined that the high LILRB4 expression group has high malignancy and poor prognosis of cancer. The degree of differentiation is an index indicating how much the morphology of a cell (alveolar epithelial cell) that is the origin of cancer is maintained, as described above. The degree of differentiation decreases from G1 to G3. Highly differentiated cancer has a morphology close to that of alveolar epithelial cells, but, as the degree of differentiation decreases, the heteromorphism increases, the malignancy generally increases, and the prognosis becomes poor. As shown in Table 3, the number of patients with the degree of differentiation G3 was significantly large in the high LILRB4 expression group. Furthermore, in the high LILRB4 expression group, the number of persons positive for vascular invasion, which is a poor prognostic factor, was also significantly large in the high LILRB4 expression group. Therefore, a plurality of poor prognostic factors was observed in the high LILRB4 expression group. From this result, it was revealed that the prognosis of cancer patients can be predicted by examining the expression of LILRB4 in the TIL region of the cancer patients.

Although a significant difference was observed between EGFR mutation and tissue type, EGFR mutation is observed in about 50% of Japanese lung adenocarcinoma patients, but is reported to be observed in 5% or less of Japanese squamous cell carcinoma patients. In this study, similar tendencies were observed for EGFR mutation and tissue type.

Similarly as in Experimental Example 4, the proportion of lung squamous cell carcinoma patients in the high LILRB4 expression group was as high as 65.3%. From this, it was revealed that the LILRB4 expression on the tumor infiltrating immune system cells in the TIL region is high in lung squamous cell carcinoma.

Next, for the above 239 cases of cancer patients, multivariate analyses were performed on five items: high LILRB4 expression relative to low LILRB4 expression; stages II to IV relative to stage I of pTNM classification; male relative to female as sex; 70 years or older relative to younger than 70 years old as age; and lung squamous cell carcinoma (SCC) relative to lung adenocarcinoma as tissue type. The results of the likelihood ratio test for recurrence-free survival rate are shown in Table 4, and the results of the Wald test for recurrence-free survival rate are shown in Table 5. The results of the likelihood ratio test for overall survival rate are shown in Table 6, and the results of the Wald test for overall survival rate are shown in Table 7.

**Table 4**

| Factor | Number of parameters | Degree of freedom | Likelihood ratio chi-square | p Value (Prob > ChiSq) |
|---|---|---|---|---|
| High LILRB4 expression | 1 | 1 | 8.95217228 | 0.0028 |
| Stage (I vs. II to IV) | 1 | 1 | 25.2922662 | < 0.001 |
| Age (70 years or older) | 1 | 1 | 2.0828834 | 0.1490 |
| Sex (male) | 1 | 1 | 9.51973147 | 0.0020 |
| Tissue type (SCC) | 1 | 1 | 4.96396455 | 0.0259 |

**Table 5**

| Factor | Number of parameters | Degree of freedom | Wald chi-square | p Value (Prob > ChiSq) |
|---|---|---|---|---|
| High LILRB4 expression | 1 | 1 | 9.21558818 | 0.0024 |
| Stage (I vs. II to IV) | 1 | 1 | 26.646702 | < 0.0001 |
| Age (70 years or older) | 1 | 1 | 2.06657165 | 0.1506 |
| Sex (male) | 1 | 1 | 8.742559 | 0.0031 |
| Tissue type (SCC) | 1 | 1 | 5.00557822 | 0.0253 |

**Table 6**

| Factor | Number of parameters | Degree of freedom | Likelihood ratio chi-square | p Value (Prob > ChiSq) |
|---|---|---|---|---|
| High LILRB4 expression | 1 | 1 | 4.11864458 | 0.0424 |
| Stage (I vs. II to IV) | 1 | 1 | 15.4795404 | < 0.0001 |
| Age (70 years or older) | 1 | 1 | 2.20486932 | 0.1376 |
| Sex (male) | 1 | 1 | 18.7124049 | < 0.001 |
| Tissue type (SCC) | 1 | 1 | 1.75471269 | 0.1853 |

**Table 7**

| Factor | Number of parameters | Degree of freedom | Wald chi-square | p Value (Prob > ChiSq) |
|---|---|---|---|---|
| High LILRB4 expression | 1 | 1 | 4.21515155 | 0.0401 |
| Stage (I vs. II to IV) | 1 | 1 | 16.0637252 | < 0.0001 |
| Age (70 years or older) | 1 | 1 | 2.16300015 | 0.1414 |
| Sex (male) | 1 | 1 | 13.3456528 | 0.0003 |
| Tissue type (SCC) | 1 | 1 | 1.76827043 | 0.1836 |

As shown in Tables 4 to 7, in both the recurrence-free survival rate and the overall survival rate, high LILRB4 expression was a poor prognostic factor independent of pTNM classification, sex, age, and tissue type.

### [Experimental Example 9] Analysis of Prognosis of Non-small Cell Lung Carcinoma Patient

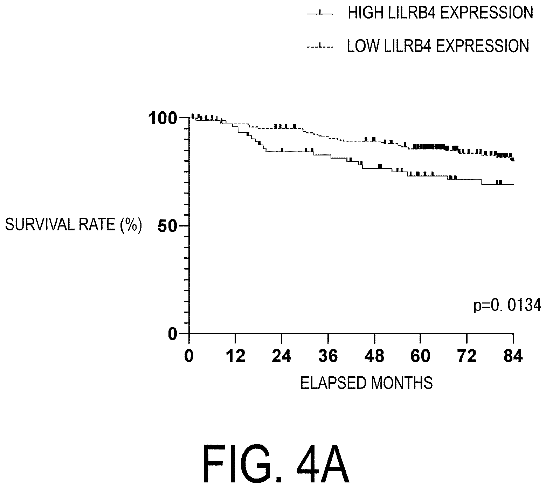

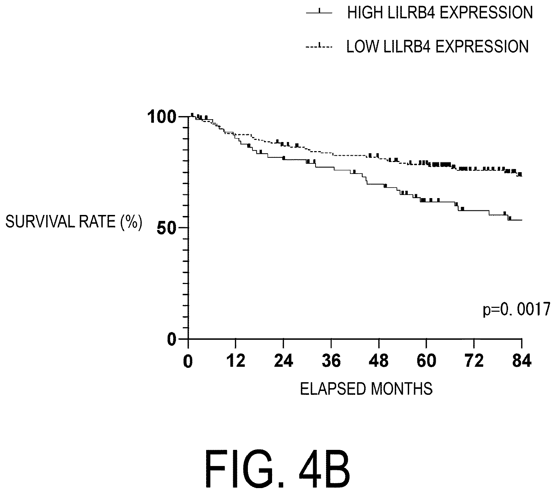

In the patients of Experimental Example 8, the high LILRB4 expression group and low expression group were examined for overall survival rate and recurrence-free survival rate. The overall survival rate is shown in FIG. 4A, and the recurrence-free survival rate is shown in FIG. 4B.

As shown in FIGS. 4A and 4B, both the overall survival rate and the recurrence-free survival rate were significantly higher in the low LILRB4 expression group. This result also showed that the prognosis of the cancer patients is poor when LILRB4 is highly expressed in the TIL region.

### [Experimental Example 10] Analysis of Prognosis of Lung Squamous Cell Carcinoma Patient

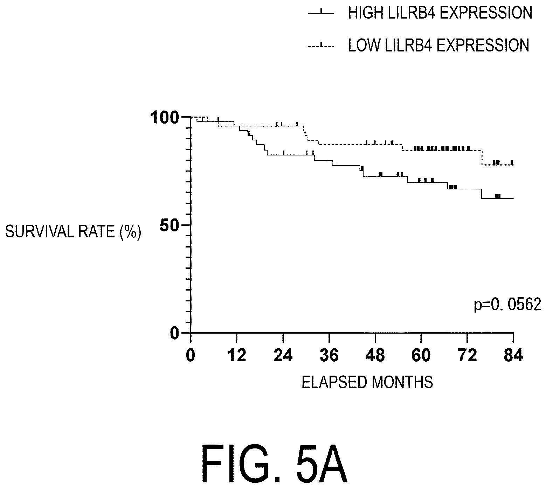

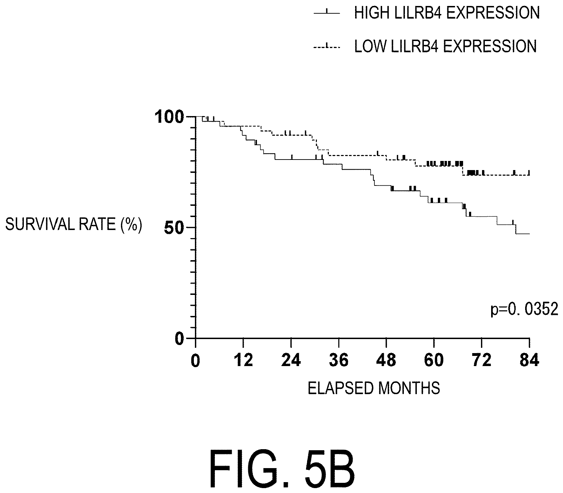

Among the 239 cases of cancer patients used in Experimental Example 8, only 97 cases of lung squamous cell carcinoma patients were used to examine the overall survival rate and recurrence-free survival rate for the high LILRB4 expression group and low expression group. The overall survival rate is shown in FIG. 5A, and the recurrence-free survival rate is shown in FIG. 5B.

As shown in FIG. 5A, the overall survival rate tended to be higher in the low LILRB4 expression group although the difference was not significant. Further, as shown in FIG. 5B, the recurrence-free survival rate was significantly higher in the low LILRB4 expression group. This result also showed that the prognosis is poor when LILRB4 is highly expressed in the TIL region also in the lung squamous cell carcinoma patients.

### Industrial Applicability

The present invention can provide a biomarker for predicting prognosis of a cancer patient, including LILRB4 or LILRB4 genes, a method for predicting prognosis of a cancer patient, a method for predicting an effect of a cancer therapeutic drug in a cancer patient, and a kit for predicting prognosis of a cancer patient.

## Claims

1. A biomarker for predicting prognosis of a cancer patient, the biomarker comprising an immunosuppressive receptor LILRB4 or an immunosuppressive receptor LILRB4 gene.

2. The biomarker according to claim 1, wherein the cancer is lung cancer.

3. A method for predicting prognosis of a cancer patient, the method comprising:
measuring an expression level of an immunosuppressive receptor LILRB4 or an immunosuppressive receptor LILRB4 gene in a biological sample of the cancer patient,
wherein
a higher expression level of the immunosuppressive receptor LILRB4 or the immunosuppressive receptor LILRB4 gene in tumor infiltrating immune system cells in the biological sample than an expression level of the immunosuppressive receptor LILRB4 or the immunosuppressive receptor LILRB4 gene in a control indicates poor prognosis of the cancer patient.

4. The method according to claim 3, wherein the cancer is lung cancer.

5. The method according to claim 3 or 4, wherein, in the measuring of the expression level of the immunosuppressive receptor LILRB4, the expression level of the immunosuppressive receptor LILRB4 is measured using an anti-immunosuppressive receptor LILRB4 monoclonal antibody or a fragment thereof.

6. The method according to claim 5, wherein
an amino acid sequence of a complementarity determining region (hereinafter, also referred to as CDR) 1 of a heavy chain variable region (hereinafter, also referred to as VH) of the anti-immunosuppressive receptor LILRB4 monoclonal antibody comprises an amino acid sequence represented by SEQ ID NO: 3,
an amino acid sequence of a CDR2 of the VH comprises an amino acid sequence represented by SEQ ID NO: 4,
an amino acid sequence of a CDR3 of the VH comprises an amino acid sequence represented by SEQ ID NO: 5,
an amino acid sequence of a CDR1 of a light chain variable region (hereinafter, also referred to as VL) comprises an amino acid sequence represented by SEQ ID NO: 6,
an amino acid sequence of a CDR2 of the VL comprises an amino acid sequence represented by SEQ ID NO: 7, and
an amino acid sequence of a CDR3 of the VL comprises an amino acid sequence represented by SEQ ID NO: 8.

7. The method according to claim 3 or 4, wherein the cancer patient is a cancer patient who has been administered a cancer therapeutic drug.

8. The method according to claim 7, wherein the cancer therapeutic drug is a substance that inhibits binding between fibronectin and the immunosuppressive receptor LILRB4.

9. The method according to claim 8, wherein the substance that inhibits binding between fibronectin and the immunosuppressive receptor LILRB4 is an anti-human immunosuppressive receptor LILRB4 antibody.

10. A method for predicting an effect, as a cancer therapeutic drug in a cancer patient, of a substance that inhibits binding between fibronectin and an immunosuppressive receptor LILRB4, the method comprising:
measuring an expression level of an immunosuppressive receptor LILRB4 or an immunosuppressive receptor LILRB4 gene in a biological sample of the cancer patient,
wherein
a higher expression level of the immunosuppressive receptor LILRB4 or the immunosuppressive receptor LILRB4 gene in tumor infiltrating immune system cells in the biological sample than an expression level of the immunosuppressive receptor LILRB4 or the immunosuppressive receptor LILRB4 gene in a control indicates that the cancer therapeutic drug has an effect on the cancer patient.

11. The method according to claim 10, wherein the cancer is lung cancer.

12. The method according to claim 10 or 11, wherein the substance that inhibits binding between fibronectin and an immunosuppressive receptor LILRB4 is an anti-human immunosuppressive receptor LILRB4 antibody.

13. The method according to claim 10 or 11, wherein, in the measuring of the expression level of the immunosuppressive receptor LILRB4, the expression level of the immunosuppressive receptor LILRB4 is measured using an anti-immunosuppressive receptor LILRB4 monoclonal antibody or a fragment thereof.

14. The method according to claim 13, wherein
an amino acid sequence of a complementarity determining region (hereinafter, also referred to as CDR) 1 of a heavy chain variable region (hereinafter, also referred to as VH) of the anti-immunosuppressive receptor LILRB4 monoclonal antibody comprises an amino acid sequence represented by SEQ ID NO: 3,
an amino acid sequence of a CDR2 of the VH comprises an amino acid sequence represented by SEQ ID NO: 4,
an amino acid sequence of a CDR3 of the VH comprises an amino acid sequence represented by SEQ ID NO: 5,
an amino acid sequence of a CDR1 of a light chain variable region (hereinafter, also referred to as VL) comprises an amino acid sequence represented by SEQ ID NO: 6,
an amino acid sequence of a CDR2 of the VL comprises an amino acid sequence represented by SEQ ID NO: 7, and
an amino acid sequence of a CDR3 of the VL comprises an amino acid sequence represented by SEQ ID NO: 8.

15. A kit for predicting prognosis of a cancer patient, the kit comprising:
a reagent for measuring an expression level of the biomarker described in claim 1 or 2.

16. The kit according to claim 15, wherein the cancer is lung cancer.

17. The kit according to claim 15, wherein the reagent for measuring an expression level of the biomarker comprises an anti-immunosuppressive receptor LILRB4 monoclonal antibody or a fragment thereof.

18. The kit according to claim 17, wherein
an amino acid sequence of a complementarity determining region (hereinafter, also referred to as CDR) 1 of a heavy chain variable region (hereinafter, also referred to as VH) of the anti-immunosuppressive receptor LILRB4 monoclonal antibody comprises an amino acid sequence represented by SEQ ID NO: 3,
an amino acid sequence of a CDR2 of the VH comprises an amino acid sequence represented by SEQ ID NO: 4,
an amino acid sequence of a CDR3 of the VH comprises an amino acid sequence represented by SEQ ID NO: 5,
an amino acid sequence of a CDR1 of a light chain variable region (hereinafter, also referred to as VL) comprises an amino acid sequence represented by SEQ ID NO: 6,
an amino acid sequence of a CDR2 of the VL comprises an amino acid sequence represented by SEQ ID NO: 7, and
an amino acid sequence of a CDR3 of the VL comprises an amino acid sequence represented by SEQ ID NO: 8.
